# EUROPEAN PATENT SPECIFICATION

(11) **EP 1 408 769 B1**
(45) Date of publication and mention of the grant of the patent: **13.01.2010**
(21) Application number: 01990426.7
(22) Date of filing: 19.12.2001
(51) Int. Cl.: A61K 9/68

(54) **A PROCESS FOR THE PREPARATION OF MEDICATED CHEWING GUMS**
VERFAHREN ZUR HERSTELLUNG VON MEDIZINISCHEM KAUGUMMI
PROCEDE DE PREPARATION DE CHEWING GUMS MEDICAMENTEUX CONTENANT DES PRINCIPES ACTIFS INSTABLES A L'HUMIDITE

(30) Priority: 22.12.2000 IT MI20002810
(43) Date of publication of application: 21.04.2004
(73) Proprietor: PIERRE FABRE MEDICAMENT, 92654 Boulogne Cédex (FR)
(72) Inventor: BADETTI, Rolando, I-20052 Monza (IT)
(74) Representative: Ahner, Francis
(86) International application number: PCT/EP2001/013473
(87) International publication number: WO 2002/051258

(56) References cited:
- EP-A- 0 407 019
- US-A- 4 150 161
- US-A- 4 514 422
- US-A- 4 737 366

## Description

The present invention concerns a process for the preparation of medicated chewing gums, particularly a process for the preparation of base gum suitable as component for medicated chewing gums containing active principles being labile to the humidity.

The base gum of chewing gums is a mixture comprising generally elastomers, resins, plasticizers, insoluble audjuvants, food antioxidants. All the base gums addressed to the preparation of chewing gums must be, of course, consistent with the rules for the direct preparations for food use, and in the specific case of the medicated chewing gums, the problems generated for the presence of active principle must be taken into consideration.

For the production of the chewing gums there are two different approaches, the first one uses heat, while the second one consists of a cold-pressure technique of powders. In such a case the base gum is therefore powdered and then mixed with one or more active principles, sweeteners, flavours and other various components in order to obtain an homogenous mixture. The obtained mixture is then passed through a tabletting machine for the production of the tablets.

The base gum powder obtained in the grinding step before being mixed with other components for the production of the finished gum is unloaded in suitable containers and let in the open air for variable times. In view of the fact that the grinding step occurred with a cold treatment and the ground gum comes out at a temperature below 0°C, the low temperature generates condensation of the humidity, present in the air, on the powder. The base gum slowly reaches the room temperature and the final humidity level has a value comprised of 1-3%, which is variable in a range being dependent on how long the ground and cold gum is let in the open air and on the humidity level which is present in the particular moment.

Until now this final humidity level value in the gum can not be further lowered according to the known techniques, thereby the medicated gums containing active principles being labile to the humidity are more difficult to be obtained. As a matter of fact a process for the production of chewing gums which are obtained by pressure and are comprised of active principles being labile to the humidity is not known. Patent Publication US 4 737 366 discloses the manufacture of medicated gums involving cooling and drying steps.

It was surprisingly found that through a post-grinding treatment and a certain dwelling time of the base gum it is possible to reduce further the amount of the humidity of the base gum.

An object of the present invention is therefore to provide a process for the preparation of the base gum which allows an active principle being labile to the humidity to be included in the final medicated gum.

The above indicated object is reached by providing the process as recited in claim 1. Further advantages of the invention are obtained through the characteristics recited in the dependent claims.

The process according to the present invention comprises the following steps:
a) podwering the base gum in a grinding chamber;
b) reducing the amount of the humidity of the ground gum,
c) mixing the obtained powder with one or more active principles and suitable additives until the mixture is becoming homogeneous; and
d) pressing the obtained mixture in to tablets of desired size;
characterized in that in step b) the ground gum is introduced in a mixer-dryer together with a dehydrated substance and rotatably placed under vacuum at a temperature of 15-20°C.

According to the present invention the humidity level of the base gum of step b) as measured with Karl Fischer apparatus is comprised between 0.01 and 0.1 %.

The term "base gum" is intended as meaning a starting product of a mixture comprising generally elastomers, resins, plasticizers, insoluble audjuvants, food antioxidants.

Advantageously the dehydrated substance is anhydrous silica precipitate in amount of 2% by weight.

According to an embodiment of the present invention the regulation of the temperature is carried out through circulation of a fluid in the cold jacket, preferably water and ethylene glycol at a temperature of 15-20°C.

Advantageously the dwelling time in the mixer-dryer is of 2-3 hours.

Preferably the active principles being labile to the humidity and mixed with the gum are selected from the group consisting of ascorbic acid, sodium ascorbate, acetylsalicilic acid, acetylcysteine.

Preferably the additives mixed with the gum are selected from the group consisting of sweeteners, flavours, lubricants, antiadherents, fillers.

The mixing step c) is preferably carried out in a rotary powder mixer, selected between biconic mixer, "V" mixer or cubic mixer.

The so obtained mixture is preferably pressed with a rotary tabletting machine which transforms into tablets of size of 8-28 mm.
Some illustrative embodiments now follow by way of example.

### Example A

### Preparation of base gum

The base gum, sold by GUM BASE S.p.A. of Lainate (Milano, Italy), is introduced in a loading hopper of a hammer mill, from which it is then passed to the grinding chamber. The cold-grinding is so carried out according to the prior art and at the outlet the ground base gum with granule sizes below 3mm is obtained.

The gum, at the outlet of the mill, is immediately sealed in a polythene bag in order to avoid the condensation of the room humidity. The gum is then introduced in a mixer-dryer together with 2% by weight of anhydrous silica precipitate. Vacuum is then carried out in the apparatus which is rotated.

A fluid (consisting of water and ethylene glycol), the temperature of which is regulated between 15 and 20°C, is contemporaneously circulated in the apparatus jacket.

The apparatus rotates and the ground gum is mixed with the silica; contemporaneously the gum takes heat from the fluid circulating in the apparatus jacket and the humidity possibly adsorbed during treatments is evaporated and taken away by the applied vacuum. After 2/3 hours of operation, the gum temperature reaches a value of 15-20°C; at this time the machine is stopped, the gum is unloading in to double polythene bag and placed in a cardboard container. Between the two bags a sachet containing silica gel as dehydrating substance, is introduced.

The so obtained humidity level in the gum is comprised between 0.01-0.1% as measured with Karl Fischer apparatus.

### Example n.1

In a "biconical" mixer the following powders are introduced:

| | | | |
|---|---|---|---|
| Base gum ground and obtained in Example A | mg | 1050 | Gum |
| Ascorbic acid | mg | 206 | Active Principle |
| Sodium ascorbate | mg | 331 | Active Principle |
| Aspartame | mg | 7 | Sweetener |
| Sorbitol | mg | 150 | Sweetener |
| Isomalt | mg | 41 | Sweetener |
| Anhydrous Silica Precipitate | mg | 40 | antiadherent |
| Talc | mg | 40 | antiadherent |
| Magnesium Stearate | mg | 30 | Lubricant |
| Powdered Orange flavour | mg | 60 | Flavourant |
| Powdered Tangerine Flavour | mg | 40 | Flavourant |
| | mg | 2000 | |

All the above ingredients are mixed for 20 minutes and the obtained powder is pressed with a tabletting machine obtaining gums of 2 g. The humidity of the tablets just produced and measured with the Karl Fischer apparatus is 0.22%. Such a result allowed a product which is stable for two years to be obtained.

### Example n.2

In a "biconical" mixer the following powders are introduced:

| | | | |
|---|---|---|---|
| Base gum ground and obtained in Example A | mg | 1065 | Gum |
| Coated Salicic acid | mg | 516 | Active Principle |
| Aspartame | mg | 6 | Sweetener |
| Potassium Acesulphame | mg | 4 | Sweetener |
| Betacyclodextrin | mg | 74 | Sweetener |
| Silica Precipitate | mg | 40 | antiadherent |
| Talc | mg | 40 | antiadherent |
| Powdered Spearmint Flavour | mg | 50 | Flavourant |
| Ammonium glycirrinizate | mg | 5 | Flavourant |
| | mg | 1800 | |

All the above ingredients are mixed for 20 minutes and the obtained powder is pressed with a tabletting machine obtaining gums of 1.8 g.

The humidity of the tablets just produced and measured with the Karl Fischer apparatus is 0.11%. Such a result allowed a product which is stable for two years to be obtained.

### Example n.3

| | | | |
|---|---|---|---|
| Base gum ground and obtained in Example A | mg | 690 | Gum |
| Acetyl cysteine | mg | 100 | Active Principle |
| Aspartame | mg | 3 | Sweetener |
| Potassium Acesulphame | mg | 2 | Sweetener |
| Betacyclodextrin | mg | 130 | Audiuvant |
| Anhydrous Silica Precipitate | mg | 35 | antiadherent |
| Talc | mg | 35 | antiadherent |
| Powdered Orange flavour | mg | 100 | Flavourant |
| Ammonium glycirinizate | mg | 5 | Flavourant |
| Magnesium stearate | mg | 30 | Lubrificant |
| Xilitol | mg | 70 | Sweetener |
| | mg | 1200 | |

All the above ingredients are mixed for 20 minutes and the obtained powder is pressed with a tabletting machine obtaining gums of 1.2 g. The humidity of the tablets just produced and measured with the Karl Fischer apparatus is 0.19%. Such a result allowed a product which is stable for two years to be obtained

## Claims

1. A process for the preparation of medicated gums comprising the step of:
a) making in powder form the base gum in a grinding chamber;
b) reducing the amount of humidity of the ground gum;
c) mixing the obtained powder with one or more active principles and suitable additives in order to obtain a homogenous mixture; and
d) pressing the obtained mixture into tablets of desired sizes,
**characterized in that** in step b) the ground gum is introduced in a mixer-dryer together with a dehydrated substance and rotatably placed under vacuum at a temperature of 15-20°C.

2. The process according to claim 1 wherein the humidity level of the base gum obtained by step b) as measured with Karl Fischer apparatus is comprised of 0.01-0.1 %.

3. The process according to claim 1 or 2 wherein the dehydrated substance is anhydrous silica precipitate.

4. The process according to anyone of the preceding claims wherein the dehydrated substance is anhydrous silica precipitate in amount of 2% by weight.

5. The process according to anyone of the preceding claims wherein the regulation of the temperature is carried out through circulation in the cold jacket of a fluid at a temperature of 15-20°C.

6. The process according to claim 5 wherein the fluid is ethylene glycol and water.

7. The process according to anyone of the preceding claims wherein the dwelling time in the mixer-dryer is of 2-3 hours.

8. The process according to anyone of the preceding claims wherein the active principles mixed with the gum are selected from the group consisting of ascorbic acid, sodium ascorbate, acetylsalicilic acid, acetylcysteine.

9. The process according to anyone of the preceding claims wherein the additives mixed with gum are selected from the group consisting of sweeteners, flavours, lubricants, antiadherents, fillers.

10. The process according to anyone of the preceding claims wherein the mixing step c) is carried out in a rotary powder mixer, preferably selected between biconic mixer, "V" mixer or cubic mixer and the pressuring step d) is carried out in a rotary tabletting machine.

## Patentansprüche

1. Verfahren zur Herstellung von medizinischen Gummis, umfassend die Schritte:
a) Herstellung des Basengummis in Pulverform in einer Mahlkammer;
b) Verringern der Feuchtigkeitsmenge des gemahlenen Gummis;
c) Mischen des erhaltenen Pulvers mit einem oder mehreren Wirkstoffen und geeigneten Zusätzen, um eine homogene Mischung zu erhalten, und
d) Pressen der erhaltenen Mischung zu Tabletten gewünschter Größen, **dadurch gekennzeichnet, dass** in Schritt b) der gemahlene Gummi zusammen mit einer dehydratisierten Substanz in einen Mischer-Trockner eingeführt und bei einer Temperatur von 15 - 20 °C drehbar unter Vakuum gesetzt wird.

2. Verfahren nach Anspruch 1, bei dem das Feuchtigkeitsniveau des durch Schritt b) erhaltenen Basengummis, wie durch eine Karl-Fischer-Apparatur gemessen, zwischen 0,01 -0,1% einschließlich liegt.

3. Verfahren nach Anspruch 1 oder 2, bei dem die dehydratisierte Substanz wasserfreies Siliciumdioxid-Präzipitat ist.

4. Verfahren nach irgendeinem der vorangehenden Ansprüche, bei dem die dehydratisierte Substanz wasserfreies Siliciumdioxid-Präzipitat in einer Menge von 2 Gew.-% ist.

5. Verfahren nach irgendeinem der vorangehenden Ansprüche, bei dem die Temperaturregulierung durch Zirkulation eines Fluids bei einer Temperatur von 15 - 20 °C im Kühlmantel durchgeführt wird.

6. Verfahren nach Anspruch 5, bei dem das Fluid Ethylenglycol und Wasser ist.

7. Verfahren nach irgendeinem der vorangehenden Ansprüche, bei dem die Verweilzeit in dem Mischer-Trockner 2 - 3 Stunden beträgt.

8. Verfahren nach irgendeinem der vorangehenden Ansprüche, bei dem die Wirkstoffe, die mit dem Gummi gemischt werden, ausgewählt sind aus der Gruppe bestehend aus Ascorbinsäure, Natriumascorbat, Acetylsalicylsäure, Acetylcystein.

9. Verfahren nach irgendeinem der vorangehenden Ansprüche, bei dem die Zusätze, die mit dem Gummi gemischt werden, ausgewählt sind aus der Gruppe bestehend aus Süßungsmitteln, Geschmacksstoffen, Gleitmitteln; Antihaftmitteln, Füllstoffen.

10. Verfahren nach irgendeinem der vorangehenden Ansprüche, bei dem der Mischschritt c) in einem Rotationspulvermischer durchgeführt wird, der vorzugsweise aus einem bikonischen Mischer, einem "V"-Mischer und einem kubischen Mischer ausgewählt ist, und der Pressschritt d) in einer Rotationstablettiermaschine durchgeführt wird.

## Revendications

1. Procédé de préparation de gommes-médicaments comprenant les étapes consistant à :
a) préparer la gomme de base sous une forme pulvérulente dans une chambre de broyage ;
b) réduire le degré d'humidité de la gomme broyée ;
c) mélanger la poudre obtenue avec un ou plusieurs principes actifs et additifs convenables afin d'obtenir un mélange homogène ; et
d) transformer le mélange obtenu par compression pour obtenir des comprimés ayant des tailles souhaitées,
**caractérisé en ce que** dans l'étape b) la gomme broyée est introduite dans un mélangeur-séchoir avec une substance déshydratée et est mise en rotation sous vide à une température de 15 à 20 °C.

2. Procédé selon la revendication 1, dans lequel le degré d'humidité de la gomme de base obtenu à l'issue de l'étape b), mesuré avec un appareil Karl Fisher, représente de 0,01 à 0,1 %.

3. Procédé selon les revendications 1 ou 2, dans lequel la substance déshydratée est un précipité de silice anhydre.

4. Procédé selon l'une quelconque des revendications précédentes, dans lequel la substance déshydratée est un précipité de silice anhydre en une quantité de 2 % en poids.

5. Procédé selon l'une quelconque des revendications précédentes, dans lequel la régulation de la température s'effectue par circulation dans la chemise froide d'un fluide à une température de 15 à 20 °C.

6. Procédé selon la revendication 5, dans lequel le fluide est un mélange d'éthylène glycol et d'eau.

7. Procédé selon l'une quelconque des revendications précédentes, dans lequel le temps de séjour dans le mélangeur-séchoir est de 2 à 3 heures.

8. Procédé selon l'une quelconque des revendications précédentes, dans lequel les principes actifs mélangés avec la gomme sont choisis dans le groupe constitué par l'acide ascorbique, l'ascorbate de sodium, l'acide acétylsalicylique, l'acétylcystéine.

9. Procédé selon l'une quelconque des revendications précédentes, dans lequel les additifs mélangés avec la gomme sont choisis dans le groupe constitué par les édulcorants, les aromatisants, les lubrifiants, les anti-adhérents, les charges.

10. Procédé selon l'une quelconque des revendications précédentes, dans lequel l'étape de mélange c) est mise en oeuvre dans un mélangeur de poudres rotatif, de préférence, choisi entre un mélangeur biconique, un mélangeur en « V » ou un mélangeur cubique et l'étape de compression d) est mise en oeuvre dans une pastilleuse rotative.
